# EUROPÄISCHE PATENTSCHRIFT

(11) **EP 1 761 483 B1**
(45) Veröffentlichungstag und Bekanntmachung des Hinweises auf die Patenterteilung: **27.10.2010**
(21) Anmeldenummer: 05761527.0
(22) Anmeldetag: 22.06.2005
(51) Int. Cl.: C07C 263/10, C07C 265/14

(54) **VERFAHREN ZUR HERSTELLUNG VON ISOCYANATEN**
METHOD FOR PRODUCING ISOCYANATES
PROCEDE POUR PRODUIRE DES ISOCYANATES

(30) Priorität: 22.06.2004 DE 102004030164
(43) Veröffentlichungstag der Anmeldung: 14.03.2007
(73) Patentinhaber: BASF SE, 67056 Ludwigshafen (DE)
(72) Erfinder: KNÖSCHE, Carsten, 67150 Niederkirchen (DE); WÖLFERT, Andreas, 74906 Bad Rappenau (DE); DAISS, Andreas, 68165 Mannheim (DE)
(86) Internationale Anmeldenummer: PCT/EP2005/006745
(87) Internationale Veröffentlichungsnummer: WO 2005/123665

(56) Entgegenhaltungen:
- DE-A1- 10 245 704
- US-A- 5 679 839

## Beschreibung

Gegenstand der Erfindung ist ein Verfahren zur Herstellung von Isocyanaten in der Gasphase. Isocyanate werden in großen Mengen hergestellt und dienen hauptsächlich als Ausgangsstoffe zur Herstellung von Polyurethanen. Ihre Herstellung erfolgt zumeist durch Umsetzung der entsprechenden Amine mit Phosgen.

Eine Möglichkeit der Herstellung von Isocyanaten ist die Umsetzung in der Gasphase. Die Vorteile dieser Verfahrensführungen liegen in einem verringerten Phosgen-Hold-Up, in der Vermeidung schwer phosgenierbarer Zwischenprodukte und erhöhten Reaktionsausbeuten. Neben einer effektiven Vermischung der Eduktströme stellen die Realisierung eines engen Verweilzeitspektrums und die Einhaltung eines engen Verweilzeitfensters wichtige Voraussetzungen für die technische Durchführbarkeit eines derartigen Prozesses dar. Diese Anforderungen können beispielsweise durch den Einsatz von turbulent betriebenen Rohrreaktoren oder durch Strömungsrohre mit Einbauten befriedigt werden.

Aus dem Stand der Technik sind verschiedene Verfahren zur Herstellung von Isocyanaten durch Umsetzung von Aminen mit Phosgen in der Gasphase bekannt. EP-A-593 334 beschreibt ein Verfahren zur Herstellung von aromatischen Diisocyanaten in der Gasphase, **dadurch gekennzeichnet, dass** die Umsetzung des Diamins mit Phosgen in einem Rohrreaktor ohne bewegte Teile und mit einer Verengung der Wände entlang der Längsachse des Rohrreaktors stattfindet. Das Verfahren ist jedoch problematisch, da die Vermischung der Edukt-ströme allein über eine Verengung der Rohrwand schlecht im Vergleich zur Anwendung eines richtigen Mischorganes funktioniert. Eine schlechte Vermischung führt üblicherweise zu einer unerwünscht hohen Feststoffbildung.

EP-A-699 657 beschreibt ein Verfahren zur Herstellung von aromatischen Diisocyanaten in der Gasphase, **dadurch gekennzeichnet, dass** die Umsetzung des zugehörigen Diamins mit dem Phosgen in einem zweizonigen Reaktor stattfindet, wobei die erste Zone, die 20 % bis 80 % des Gesamtreaktorvolumens ausmacht, ideal vermischt ist und die zweite Zone, die 80 % bis 20 % des Gesamtreaktorvolumens ausmacht, durch eine Kolbenströmung charakterisiert werden kann. Weil jedoch mindestens 20 % des Reaktionsvolumens ideal rückvermischt sind, resultiert eine ungleichmäßige Verweilzeitverteilung, die zu einer unerwünscht erhöhten Feststoffbildung führen kann.

EP-A-289 840 beschreibt die Herstellung von Diisocyanaten durch Gasphasenphosgenierung, wobei die Herstellung erfindungsgemäß in einer turbulenten Strömung bei Temperaturen zwischen 200°C und 600°C in einem zylindrischen Raum ohne bewegte Teile stattfindet. Durch den Verzicht auf bewegte Teile wird die Gefahr eines Phosgenaustrittes reduziert. Durch die turbulente Strömung im zylindrischen Raum (Rohr) wird, wenn man von Fluidelementen in Wandnähe absieht, eine gute Strömungsgleichverteilung im Rohr und damit eine enge Verweilzeitverteilung erreicht, die, wie in EP-A-570 799 beschrieben, zu einer Verringerung der Feststoffbildung führen kann.

EP-A-570 799 betrifft ein Verfahren zur Herstellung von aromatischen Diisocyanaten in der Gasphase, **dadurch gekennzeichnet, dass** die Umsetzung des zugehörigen Diamins mit dem Phosgen in einem Rohrreaktor oberhalb der Siedetemperatur des Diamins innerhalb einer mittleren Kontaktzeit von 0,5 bis 5 Sekunden durchgeführt wird. Wie in der Schrift beschrieben ist, führen zu lange als auch zu kurze Reaktionszeiten zu einer unerwünschten Feststoffbildung. Es wird daher ein Verfahren offenbart, bei dem die mittlere Abweichung von der mittleren Kontaktzeit weniger als 6 % beträgt. Die Einhaltung dieser Kontaktzeit wird dadurch erreicht, dass die Reaktion in einer Rohrströmung durchgeführt wird, die entweder durch eine Reynolds-Zahl von oberhalb 4.000 oder eine Bodenstein-Zahl von oberhalb 100 charakterisiert wird.

EP-A-749 958 beschreibt ein Verfahren zur Herstellung von Triisocyanate durch Gasphasenphosgenierung von (cyclo)aliphatischen Triaminen mit drei primären Aminogruppen, **dadurch gekennzeichnet, dass** man das Triamin und das Phosgen kontinuierlich in einem auf 200°C bis 600°C erhitzten, zylindrischen Reaktionsraum mit einer Strömungsgeschwindigkeit von mindestens 3 m/s miteinander zur Reaktion bringt.

EP-A-928 785 beschreibt die Verwendung von Mikrostrukturmischern zur Phosgenierung von Aminen in der Gasphase. Nachteilig bei der Verwendung von Mikromischern ist, dass bereits kleinste Feststoffmengen, deren Entstehung bei der Synthese der Isocyanate nicht vollständig auszuschliessen ist, zum Verstopfen des Mischers führen können, was die zeitliche Verfügbarkeit der Phosgenierungsanlage herabsetzt.

In allen Fällen ist es jedoch notwendig, nach Erreichen einer optimalen Reaktionszeit die Reaktion effektiv abzubrechen, um die Bildung von Feststoffen durch Folgereaktionen des Isocyanats zu verhindern.

DE 10245704 A1 beschreibt das rasche Abkühlen eines Reaktionsgemisches, das mindestens aus einem Isocyanat, Phosgen und Chlorwasserstoff besteht, in einer Quenchzone. Die Quenchzone besteht dabei aus mindestens 2 Düsenköpfen, die ihrerseits mindestens jeweils 2 Einzeldüsen enthalten. In der Quenchzone wird das Reaktionsgas mit den versprühten Flüssigkeitströpfchen vermischt. Durch die Verdampfung der Flüssigkeit wird die Temperatur des Gasgemisches schnell abgesenkt, so dass der Verlust des Isocyanatwertprodukts infolge hoher Temperaturen vermindert wird. Ferner wird durch die Düsenanordnung ein frühzeitiger Kontakt des heißen Reaktionsgases mit den Quenchzonenwandungen unterdrückt, so dass die Bildung von Belägen an den Oberflächen reduziert wird.

Nachteilig bei dem beschriebenen Verfahren sind die Quenchzeiten von 0,2 bis 3,0 s, die zu einem deutlichen, vermeidbaren Verlust an Isocyanat führen.

Aufgabe der Erfindung war es, ein Verfahren zur Herstellung von Isocyanaten in der Gasphase zu entwickeln, bei dem nach Erreichen der optimalen Verweilzeit die Reaktion innerhalb ausreichend kurzer Zeiten gestoppt wird und eine einfache Abtrennung des Isocyanats von den übrigen Bestandteilen des Reaktionsgemisches erreicht werden kann.

Die Aufgabe konnte dadurch gelöst werden, dass die Umsetzung in einer Reaktionszone durchgeführt wird und das Reaktionsgemisch zum Abbruch der Reaktion durch eine Zone geführt wird, in der eine Flüssigkeit eingedüst wird. Dabei befindet sich zwischen der Reaktionszone und der Zone, in der der Reaktionsabbruch herbeigeführt wird, ein Bereich mit reduziertem Strömungsquerschnitt.

Als Reaktionszone können ein Rohrreaktoren, Strömungsrohre mit oder ohne Einbauten oder Plattenreaktoren eingesetzt werden.

Gegenstand der Erfindung ist demzufolge ein Verfahren zur Herstellung von Isocyanaten durch Umsetzung von Aminen mit Phosgen in der Gasphase in einer Reaktionszone, wobei das Reaktionsgemisch zum Abbruch der Reaktion durch eine Zone geführt wird, in der eine Flüssigkeit eingedüst wird, **dadurch gekennzeichnet, dass** das Reaktionsgemisch zwischen der Reaktionszone und der Zone, in der die Flüssigkeit eingedüst wird, durch eine Zone geführt wird, die einen reduzierten Strömungsquerschnitt aufweist.

Die Verengung des Strömungsquerschnitts ist dabei so gewählt, dass das Reaktionsgas beim Verlassen der Verengung einerseits merklich abgekühlt ist und andererseits eine hohe Strömungsgeschwindigkeit besitzt, die eine effektive Sekundärzerstäubung der Quenchflüssigkeit bewirkt. Beide Anforderungen können dadurch erreicht werden, dass die Machzahl der Strömung in der Verengung 0.1 bis 1.0, bevorzugt 0.2 bis 1.0 besonders bevorzugt 0.3 bis 1.0 beträgt. Unter der Machzahl versteht man hierbei die lokale Strömungsgeschwindigkeit bezogen auf die lokale Schallgeschwindigkeit des Reaktionsgemisches. Aus der Anforderung an die Machzahl ergibt sich bei vorgegebenem Massenstrom direkt die Größe des engsten Querschnitts. Das Verhältnis von Strömungsquerschnitt in der Verengung zum Strömungsquerschnitt in der Reaktionszone beträgt 1 / 1.2 bis 1 / 10 bevorzugt 1 / 2 bis 1 / 10 besonders bevorzugt 1 / 3 bis 1 / 10.Die Zone, in der eine Flüssigkeit eingedüst wird, wird im folgenden auch als Quenchzone bezeichnet, das Eindüsen der Flüssigkeit wird als Quench bezeichnet.

In dieser Quenchzone wird das Reaktionsgemisch, das im wesentlichen aus den Isocyanaten, Phosgen und Chlorwasserstoff besteht, intensiv mit der eingedüsten Flüssigkeit vermischt. Die Vermischung erfolgt derart, dass die Temperatur des Reaktionsgemisches ausgehend von 250 bis 500°C um 50 bis 300°C, vorzugsweise 100 bis 250°C abgesenkt wird und das im Reaktionsgemisch enthaltene Isocyanat durch Kondensation vollständig oder teilweise in die eingedüsten Flüssigkeitströpfchen übergeht, während das Phosgen und der Chlorwasserstoff im wesentlichen vollständig in der Gasphase verbleiben.

Der Anteil des im gasförmigen Reaktionsgemisch enthaltenen Isocyanats, das in der Quenchzone in die Flüssigphase übergeht, beträgt dabei vorzugsweise 20 bis 100 Gew.-%, besonders bevorzugt 50 bis 100 Gew.-% und insbesondere 70 bis 100 Gew.-%, bezogen auf das im Reaktionsgemisch enthaltene Isocyanat.

Das Reaktionsgemisch durchströmt die Quenchzone vorzugsweise von oben nach unten. Unterhalb der Quenchzone ist ein Sammelbehälter angeordnet, in dem die Flüssigphase abgeschieden, gesammelt und, über einen Auslass aus dem Reaktionsraum entfernt und anschließend aufgearbeitet wird. Die verbleibende Gasphase wird über einen zweiten Auslass aus dem Reaktionsraum entfernt und ebenfalls aufgearbeitet.

Die Flüssigkeitströpfchen werden mittels Ein- oder Zweistoffzerstäuberdüsen, vorzugsweise Einstoffzerstäuberdüsen, erzeugt und besitzen vorzugsweise einen Sauter-Durchmesser d₂₃ von 5 bis 5000 µm, besonders bevorzugt 5 bis 500 µm und insbesondere 5 bis 250 µm. Der Sauterdurchmesser d₂₃ beschreibt bis auf einen konstanten Faktor das Verhältnis von Tropfenvolumen zu Tropfenoberfläche (K. Schwister: Taschenbuch der Verfahrenstechnik, Fachbuchverlag Leipzig, Carl Hanser Verlag 2003) und ist somit die für den Quenchprozess wesentliche Kenngröße der erzeugten Tropfengrößenverteilung.

Die Zerstäuberdüsen erzeugen je nach Ausführungsform einen Sprühkegelwinkel von 10 bis 140°, bevorzugt von 10 bis 120°, besonders bevorzugt von 10° bis 100°.

Erfindungsgemäß befindet sich zwischen der Reaktionszone und der Quenchzone eine Querschnittsverengung, durch die eine Entspannung, verbunden mit einer Konzentrationserniedrigung, der Reaktanten und erste Temperaturabsenkung des Reaktionsgases erzielt wird. Ferner bewirkt der aus der Querschnittverengung mit sehr hoher Geschwindigkeit austretende Reaktionsgasstrom beim Zusammentreffen mit dem Quenchflüssigkeitsspray eine Sekundarzerstäubung der Quenchflüssigkeit, so dass das Spray eine besonders große spezifische Oberfläche besitzt. Auf Grund der großen spezifischen Oberfläche und der hohen Relativgeschwindigkeiten zwischen Reaktionsgas und Quenchflüssigkeit werden der Stoff- und Wärmeaustausch zwischen Reaktionsgas und Quenchflüssigkeit intensiviert. Damit werden die für das Abkühlen des Reaktionsgemisches benötigten Kontaktzeiten stark verringert und der Verlust von Isocyanatwertprodukt infolge Weiterreaktion zu Nebenprodukten minimiert.

Der freie Strömungsquerschnitt in der Quenchzone beträgt, bezogen auf den freien Strömungsquerschnitt in der Reaktionszone 5 / 1 bis 1 / 2, bevorzugt 4 / 1 bis 1 / 1, besonders bevorzugt 3 / 1 bis 1 / 1.

Die Flüssigkeit, die über die Zerstäuberdüsen eingedüst wird, muss eine gute Löslichkeit von Isocyanaten aufweisen. Vorzugsweise werden organische Lösungsmittel eingesetzt. Insbesondere eingesetzt werden aromatische Lösungsmittel, die mit Halogenatomen substituiert sein können. Beispiele für derartige Flüssigkeiten sind Toluol, Benzol, Nitrobenzol, Anisol, Chlorbenzol, Dichlorbenzol (otho, para), Trichlorbenzol, Xylol, Hexan, Diethylisophthalat (DEIP), Tetrahydrofuran (THF), Dimethylformamid (DMF) und deren Gemische.

In einer besonderen Ausführungsform des erfindungsgemäßen Verfahrens handelt es sich bei der eingedüsten Flüssigkeit um ein Gemisch aus Isocyanaten , ein Gemisch aus Isocyanaten und Lösungsmittel oder um Isocyanat, wobei die jeweils verwendete Quenchflüssigkeit Anteile an Leichtsieder wie HCl und Phosgen aufweisen kann. Vorzugsweise wird dabei das Isocyanat eingesetzt, das bei dem jeweiligen Verfahren hergestellt wird. Da durch die Temperatursenkung in der Quenchzone die Reaktion zum Stillstand kommt, können Nebenreaktionen mit den eingedüsten Isocyanaten ausgeschlossen werden. Der Vorteil dieser Ausführungsform liegt insbesondere darin, dass auf eine Abtrennung des Lösungsmittels verzichtet werden kann.

Die Temperatur der eingedüsten Flüssigkeit liegt vorzugsweise bei 0 bis 300°C, besonders bevorzugt bei 50 bis 250°C und insbesondere bei 70 bis 200°C, so dass bei der eingedüsten Flüssigkeitsmenge die gewünschte Abkühlung und Kondensation des Isocyanates erreicht wird.

Die Geschwindigkeit des Reaktionsgases in der Quenchzone ist vorzugsweise größer als1 m/s, besonders bevorzugt größer als 10 m/s und insbesondere größer als 20 m/s.

Um eine schnelle Abkühlung der gasförmigen Reaktionsmischung in der Quenchzone und einen schnellen Übergang des Isocyanats in die Flüssigphase zu erreichen, müssen die Tröpfchen der eingedüsten Flüssigkeit sehr schnell über den gesamten Strömungsquerschnitt des Reaktionsgases fein verteilt werden. Die gewünschte Temperaturabsenkung und der gewünschte Übergang des Isocyanats in die Tröpfchen wird dabei vorzugsweise in 10⁻⁴ bis 10 Sekunden, besonders bevorzugt in 5x10⁻⁴ bis 1 Sekunden und insbesondere in 0,001 bis 0,2 Sekunden durchgeführt. Die obigen Zeiten sind dabei definiert als der Zeitraum zwischen dem Eintritt des Reaktionsgases in den Quenchbereich und dem Zeitpunkt, zu dem das Reaktionsgas noch 10 % von der adiabaten Endtemperatur des Gemisches aus Reaktionsgas und Tropfen abweicht. Durch die gewählten Zeiträume kann ein Verlust von Isocyanat durch Neben- bzw. Weiterreaktionen praktisch vollständig vermieden werden.

Das Masseverhältnis von eingedüster Flüssigkeitsmenge zur Menge der gasförmigen Reaktionsmischung beträgt vorzugsweise 100 : 1 bis 1 : 10, besonders bevorzugt 50 : 1 bis 1 : 5 und insbesondere 10 : 1 bis 1 : 2.

Die Umsetzung des Amins mit dem Phosgen in der Gasphase kann unter den bekannten Bedingungen erfolgen.

Die Vermischung der Reaktionskomponenten Amin und Phosgen kann vor oder im Reaktor erfolgen. So ist es möglich, dem Reaktor eine Mischeinheit, beispielsweise eine Düse, vorzuschalten, wodurch bereits ein gemischter Gasstrom, enthaltend Phosgen und Amin, in den Reaktor gelangt.

In einer Ausführungsform des erfindungsgemäßen Verfahrens wird zunächst der Phosgenstrom mittels eines Verteilelements auf die gesamte Breite des Reaktors möglichst homogen verteilt. Die Zufuhr des Aminstroms erfolgt am Anfang des Reaktors, hier ist ein Verteilerkanal mit Löchern oder Mischdüsen im Reaktionskanal eingebracht, wobei dieser Verteilerkanal bevorzugt über die gesamte Breite des Reaktors reicht. Aus den Löchern oder Mischdüsen wird das Amin, das gegebenenfalls mit einem Inertmedium vermischt ist, dem Phosgenstrom zugeführt.

Bei dem Inertmedium handelt es sich um ein Medium, das bei der Reaktionstemperatur gasförmig vorliegt und nicht mit den Edukten reagiert. Beispielsweise können Stickstoff, Edelgase wie Helium oder Argon oder Aromaten wie Chlorbenzol, Dichlorbenzol oder Xylol verwendet werden. Bevorzugt wird Stickstoff als Inertmedium verwendet.

Für das erfindungsgemäße Verfahren können primäre Amine verwendet werden, die bevorzugt ohne Zersetzung in die Gasphase überführt werden können. Besonders geeignet sind hier Amine, insbesondere Diamine, auf Basis von aliphatischen oder cycloaliphatischen Kohlenwasserstoffen mit 1 bis 15 Kohlenstoffatomen. Beispiele hierfür sind 1,6-Diaminohexan, 1-Amino-3,3,5-trimethyl-5-aminomethylcyclohexan (IPDA) und 4,4'-Diaminodicyclohexylmethan. Bevorzugt verwendet wird 1,6-Diaminohexan (HDA).

Für das erfindungsgemäße Verfahren können auch aromatische Amine verwendet werden, die bevorzugt ohne Zersetzung in die Gasphase überführt werden können. Beispiele für bevorzugte aromatische Amine sind Toluylendiamin (TDA), vorzugsweise 2,4- oder 2,6-Isomere oder Gemische davon, Diaminobenzol, Napthyldiamin (NDA) und 2,4'- oder 4,4'-Methylen(diphenylamin) (MDA) oder Isomerengemische davon. Beim erfindungsgemäßen Verfahren ist es vorteilhaft, Phosgen im Überschuss zu den Aminogruppen einzusetzen. Üblicherweise liegt ein molares Verhältnis von Phosgen zu Aminogruppen von 1,1 : 1 bis 20 : 1, bevorzugt von 1,2 : 1 bis 5 : 1 vor.

Zur Durchführung des erfindungsgemäßen Verfahrens kann es vorteilhaft sein, die Ströme der Reaktanten vor dem Vermischen vorzuwärmen, üblicherweise auf Temperaturen von 100 bis 600°C, bevorzugt von 200 bis 500°C. Die Umsetzung im Reaktionskanal findet üblicherweise bei einer Temperatur von 150 bis 600°C, bevorzugt von 250 bis 500°C statt. Das erfindungsgemäße Verfahren wird bevorzugt kontinuierlich durchgeführt.

In einer bevorzugten Ausführungsform werden die Abmessungen des Reaktors und die Strömungsgeschwindigkeiten so bemessen, dass eine turbulente Strömung, d.h. eine Strömung mit einer Reynolds-Zahl von mindestens 2300, bevorzugt mindestens 2700, vorliegt, wobei die Reynolds-Zahl mit dem hydraulischen Durchmesser des Reaktors gebildet wird. Die Reynoldszahl legt hierbei das Strömungsregime und damit die Verweilzeitverteilung im Reaktionsrohr fest (H. Schlichting: Grenzschichttheorie, Verlag G. Braun, 1982; M. Baerns: Chemische Reaktionstechnik, Georg Thieme Verlag Stuttgart, 1992). Bevorzugt durchlaufen die gasförmigen Reaktionspartner den Reaktor mit einer Strömungsgeschwindigkeit von 20 bis 150 Meter/Sekunde, bevorzugt von 30 bis 100 Meter/Sekunde.

Im allgemeinen beträgt bei dem erfindungsgemäßen Verfahren die mittlere Kontaktzeit 0,05 bis 5 Sekunden, bevorzugt von 0,06 bis 1, besonders bevorzugt 0,1 bis 0,45 Sekunden. Unter mittlerer Kontaktzeit wird die Zeitspanne von Beginn der Vermischung der Edukte bis zum Abbruch der Reaktion durch den Quench verstanden. In einer bevorzugten Ausführungsform ist die Strömung im erfindungsgemäßen Verfahren durch eine Bodenstein-Zahl von mehr als 10, bevorzugt mehr als 100 und besonders bevorzugt von mehr als 500 charakterisiert. Die Bodensteinzahl ist dabei ein Maß für den Rückvermischungsgrad des Strömungsapparates. Mit steigender Bodensteinzahl nimmt die Rückvermischung ab (M. Baerns: Chemische Reaktionstechnik, Georg Thieme Verlag Stuttgart, 1992)

Wie oben ausgeführt, wird am Ende des Reaktors, der ein turbulent betriebener Rohrreaktor, ein Strömungsrohr mit Einbauten oder ein Plattenreaktor sein kann, eine Quenchzone angeordnet. Die aus der Quenchzone entnommene Flüssigphase und Gasphase werden aufgearbeitet. Bei Verwendung eines Lösungsmittels als zerstäubte Flüssigkeit erfolgt eine Trennung von Isocyanat und Lösungsmittel, zumeist mittels Destillation. Die Gasphase, die im wesentlichen Phosgen, Chlorwasserstoff und gegebenenfalls nicht abgetrenntes Isocyanat enthält, kann ebenfalls, vorzugsweise durch Destillation oder Adsorption, in ihre Bestandteile zerlegt werden, wobei das Phosgen wieder der Reaktion zugeführt werden kann und der Chlorwasserstoff entweder für weitere chemische Reaktionen genutzt, zu Salzsäure weiterverarbeitet werden oder wieder in Chlor und Wasserstoff gespalten werden kann.

Die Erfindung soll an den nachstehenden Beispielen näher erläutert werden.

### Beispiel 1:

In einem Rohrreaktor (Durchmesser 8 mm) mit vorgeschaltendem Mischorgan wurden 67.5 kg/h Reaktionsgas erzeugt, das Toluoldiisocyanatisomere, Phosgen und Salzsäure enthielt. Das Reaktionsgas wurde dann über eine Querschnittsverengung mit einem Durchmesser von 3.0 mm der Quenchzone zugeführt. Dabei betrug die Machzahl im engsten Querschnitt etwa 0.85. In der Quenchzone befanden sich zwei einzelne Einstoffdüsen mit einem Sprühkegelöffnungswinkel von 80°. Die Düsen erzeugten dabei Tropfen mit einem Sauterdurchmesser von ca. 100 µm. Die eingedüste Flüssigkeitsmenge betrug 100 kg/h. Die eingedüste Quenchflüssigkeit bestand aus Monochlorbenzol. Die Temperatur des Reaktionsgases beim Eintritt in die Quenchzone betrug 363°C und der Druck des Gases 6.8 bar. Die Eintrittstemperatur der Quenchflüssigkeit betrug 100°C, die Austrittsgeschwindigkeit der Flüssigkeitströpfchen aus der Sprühdüse betrug ca. 50 m/s. Die Aufenthaltzeit des Reaktionsgases in der Quenchzone betrug ca. 0.01 Sekunden. Dabei fiel die Temperatur des Quenchgases auf 156°C ab. Die gewünschte Temperaturabsenkung erfolgte damit in weniger als 0.01 Sekunden. Die Toluoldiisocyanatmenge im Reaktionsgasgemisch nahm um 80 % gegenüber der Eingangskonzentration in der Quenchzone ab.

## Patentansprüche

1. Verfahren zur Herstellung von Isocyanaten durch Umsetzung von Aminen mit Phosgen in der Gasphase in einer Reaktionszone, wobei das Reaktionsgemisch zum Abbruch der Reaktion durch eine Zone geführt wird, in der eine Flüssigkeit eingedüst wird, **dadurch gekennzeichnet, dass** das Reaktionsgemisch zwischen der Reaktionszone und der Zone, in der die Flüssigkeit eingedüst wird, durch eine Zone geführt wird, die einen reduzierten Strömungsquerschnitt aufweist.

2. Verfahren nach Anspruch 1, **dadurch gekennzeichnet, dass** als Reaktionszone Rohrreaktoren, Strömungsrohre mit oder ohne Einbauten oder Plattenreaktoren eingesetzt werden.

3. Verfahren nach Anspruch 1, **dadurch gekennzeichnet, dass** das Verhältnis von Strömungsquerschnitt in der Verengung zum Strömungsquerschnitt in der Reaktionszone 1 / 1.2 bis 1 / 10 beträgt.

4. Verfahren nach Anspruch 1, **dadurch gekennzeichnet, dass** das Verhältnis von Strömungsquerschnitt in der Verengung zum Strömungsquerschnitt in der Reaktionszone 1 / 2 bis 1 / 10 beträgt.

5. Verfahren nach Anspruch 1, **dadurch gekennzeichnet, dass** das Verhältnis von Strömungsquerschnitt in der Verengung zum Strömungsquerschnitt in der Reaktionszone 1 / 3 bis 1 / 10 beträgt.

6. Verfahren nach Anspruch 1, **dadurch gekennzeichnet, dass** die eingedüsten Flüssigkeitstropfen einen Sauter-Durchmesser von 5 bis 5000 µm aufweisen.

7. Verfahren nach Anspruch 1, **dadurch gekennzeichnet, dass** die eingedüsten Flüssigkeitstropfen einen Sauter-Durchmesser von 5 bis 500 µm aufweisen.

8. Verfahren nach Anspruch 1, **dadurch gekennzeichnet, dass** die eingedüsten Flüssigkeitstropfen einen Sauter-Durchmesser von 5 bis 250 µm aufweisen.

9. Verfahren nach Anspruch 1, **dadurch gekennzeichnet, dass** die Temperatur der eingedüsten Flüssigkeit bei 0 bis 300°C liegt.

10. Verfahren nach Anspruch 1, **dadurch gekennzeichnet, dass** die Temperatur der eingedüsten Flüssigkeit bei 50 bis 250°C liegt.

11. Verfahren nach Anspruch 1, **dadurch gekennzeichnet, dass** die Temperatur der eingedüsten Flüssigkeit bei 70 bis 200°C liegt.

12. Verfahren nach Anspruch 1, **dadurch gekennzeichnet, dass** die eingedüste Flüssigkeit ein organisches Lösungsmittel ist.

13. Verfahren nach Anspruch 1, **dadurch gekennzeichnet, dass** die eingedüste Flüssigkeit aromatische Lösungsmittel, die mit Halogenatomen substituiert sein können, sind.

14. Verfahren nach Anspruch 1, **dadurch gekennzeichnet, dass** die eingedüste Flüssigkeit ein Isocyanat ist.

## Claims

1. A process for preparing isocyanates by reacting amines with phosgene in the gas phase in a reaction zone, with the reaction mixture being passed through a zone into which a liquid is sprayed to stop the reaction, wherein the reaction mixture is passed through a zone having a reduced flow cross section between the reaction zone and the zone into which the liquid is sprayed.

2. The process according to claim 1, wherein tube reactors, flow tubes with or without internals or plate reactors are used as reaction zone.

3. The process according to claim 1, wherein the ratio of the flow cross section in the constriction to the flow cross section in the reaction zone is from 1 / 1.2 to 1 / 10.

4. The process according to claim 1, wherein the ratio of the flow cross section in the constriction to the flow cross section in the reaction zone is from 1 / 2 to 1 / 10.

5. The process according to claim 1, wherein the ratio of the flow cross section in the constriction to the flow cross section in the reaction zone is from 1 / 3 to 1 / 10.

6. The process according to claim 1, wherein the liquid droplets sprayed in have a Sauter diameter of from 5 to 5000 µm.

7. The process according to claim 1, wherein the liquid droplets sprayed in have a Sauter diameter of from 5 to 500 µm.

8. The process according to claim 1, wherein the liquid droplets sprayed in have a Sauter diameter of from 5 to 250 µm.

9. The process according to claim 1, wherein the temperature of the liquid sprayed in is from 0 to 300°C.

10. The process according to claim 1, wherein the temperature of the liquid sprayed in is from 50 to 250°C.

11. The process according to claim 1, wherein the temperature of the liquid sprayed in is from 70 to 200°C.

12. The process according to claim 1, wherein the liquid sprayed in is an organic solvent.

13. The process according to claim 1, wherein the liquid sprayed in is an aromatic solvent which may be substituted by halogen atoms.

14. The process according to claim 1, wherein the liquid sprayed in is an isocyanate.

## Revendications

1. Procédé de fabrication d'isocyanates par réaction d'amines avec du phosgène en phase gazeuse dans une zone de réaction, le mélange réactionnel traversant une zone dans laquelle un liquide est injecté pour stopper la réaction, **caractérisé en ce que** le mélange réactionnel traverse une zone qui comporte une section d'écoulement réduite entre la zone de réaction et la zone dans laquelle le liquide est injecté.

2. Procédé selon la revendication 1, **caractérisé en ce que** des réacteurs tubulaires, des tubes d'écoulement avec ou sans composants internes ou des réacteurs à plaques sont utilisés en tant que zone de réaction.

3. Procédé selon la revendication 1, **caractérisé en ce que** le rapport entre la section d'écoulement dans le rétrécissement et la section d'écoulement dans la zone de réaction est de 1/1,2 à 1/10.

4. Procédé selon la revendication 1, **caractérisé en ce que** le rapport entre la section d'écoulement dans le rétrécissement et la section d'écoulement dans la zone de réaction est de 1/2 à 1/10.

5. Procédé selon la revendication 1, **caractérisé en ce que** le rapport entre la section d'écoulement dans le rétrécissement et la section d'écoulement dans la zone de réaction est de 1/3 à 1/10.

6. Procédé selon la revendication 1, **caractérisé en ce que** les gouttes de liquide injectées présentent un diamètre de Sauter de 5 à 5 000 µm.

7. Procédé selon la revendication 1, **caractérisé en ce que** les gouttes de liquide injectées présentent un diamètre de Sauter de 5 à 500 µm.

8. Procédé selon la revendication 1, **caractérisé en ce que** les gouttes de liquide injectées présentent un diamètre de Sauter de 5 à 250 µm.

9. Procédé selon la revendication 1, **caractérisé en ce que** la température du liquide injecté est de 0 à 300 °C.

10. Procédé selon la revendication 1, **caractérisé en ce que** la température du liquide injecté est de 50 à 250 °C.

11. Procédé selon la revendication 1, **caractérisé en ce que** la température du liquide injecté est de 70 à 200 °C.

12. Procédé selon la revendication 1, **caractérisé en ce que** le liquide injecté est un solvant organique.

13. Procédé selon la revendication 1, **caractérisé en ce que** les liquides injectés sont des solvants aromatiques, qui peuvent être substitués avec des atomes d'halogène.

14. Procédé selon la revendication 1, **caractérisé en ce que** le liquide injecté est un isocyanate.
